(19) Europäisches Patentamt
European Patent Office
Office européen des brevets

(11) Publication number: **0 233 476 B1**

(12) # EUROPEAN PATENT SPECIFICATION

(45) Date of publication of patent specification: **23.12.92**

(51) Int. Cl.5: **C08F 4/32**, C08F 2/44, C08K 5/34, C08L 67/06

(21) Application number: **87100529.4**

(22) Date of filing: **16.01.87**

(54) **Peroxide free radical initiators containing hindered amine light stabilizer groups.**

(30) Priority: **19.02.86 US 831393**

(43) Date of publication of application:
**26.08.87 Bulletin 87/35**

(45) Publication of the grant of the patent:
**23.12.92 Bulletin 92/52**

(84) Designated Contracting States:
**BE CH DE FR GB IT LI NL**

(56) References cited:
**EP-A- 0 000 405**
**EP-A- 0 056 699**
**FR-A- 2 010 740**
**US-A- 4 042 773**

(73) Proprietor: **ATOCHEM NORTH AMERICA, INC. (a Pennsylvania corp.)**
**Three Parkway**
**Philadelphia Pennsylvania 19102(US)**

(72) Inventor: **Myers, Terry N.**
**780 Maple Road**
**Williamsville New York 14221(US)**

(74) Representative: **Kraus, Walter, Dr. et al**
**Patentanwälte Kraus, Weisert & Partner**
**Thomas-Wimmer-Ring 15**
**W-8000 München 22(DE)**

EP 0 233 476 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to compounds which are free radial initiators as well as hindered amine light stabilizers.

It is well known that many ethylenically unsaturated monomers are polymerized by the use of free radical initiators, i.e., those having peroxide groups. It is also well known that many of the polymers resulting from the polymerization of such monomers are subject to degradation by ultraviolet light, and, therefore, require the presence of an ultraviolet light stabilizer to extend the useful life of such polymers. Normally, such stabilizers are added to the polymer by methods such as milling or other methods of physical mixing. In more recent times techniques of copolymerization have been employed by means of which unsaturated derivatives of certain ultraviolet light stabilizing compounds have been copolymerized with vinyl monomers to form a light stabilized polymer. The method of physically mixing the stabilizer and the polymer has always been unsatisfactory because the resulting two-phase system in incompatible. The stabilizing compound inevitably migrates to the surface of the polymer and becomes separated from the polymer by evaporation, leaching, or erosion.

The copolymerization technique is much more satisfactory than physically blending because it provides a chemically bound stabilizer which is not removed by physical processes. This method, however, has many inherent disadvantages because of the chemical equilibria involved in copolymerization reactions. The comonomer providing the stabilizer component must have its reactivity balanced against that of the principal comonomer and the concentrations of these two comonomers adjusted accordingly in order to produce a product having the desired amount of stabilizer. The copolymerization technique also normally produces a product having much more stabilizer incorporated into the polymer than is necessary, and this increases the cost of the final product markedly. Furthermore, many of the stabilizer comonomers have a tendency to homopolymerize rather than to copolymerize and thereby result in a product lacking homogeneity.

Hindered amine light stabilizers (hereinafter referred to as HALS) are a class of compounds known to prevent and/or retard the degradation of polymers in which they are incorporated. Many patents on HALS additives and monomer are in the prior art (e.g., US-A-4,336,183). The HALS compounds of the prior art suffer from the disadvantages stated above for UV stabilizers in general. The low molecular weight HALS have an added problem in that they are water soluble which prohibits their use because they are so readily leached from the polymer substrate upon exposure to moisture. Most patents issued on HALS disclose methods for making high molecular weight HALS which are less susceptible to leaching. This increase in molecular weight introduces problems of compatability with the polymer to be stabilized and enhancement of the loss of the HALS additive by exudation. Also, increasing the molecular weight may produce adverse effect in cost or physical properties, since a greater amount of additive is necessary to assure the proper level of hindered amine functionality (which may represent only a small part of the high molecular weight additive).

A specific class of HALS peroxides known in the patent literature is perketals bearing a cyclic HALS moiety (EP-A-0056699) and ammonium salts thereof (US-A-No. 4,499,273). These patents disclose the use of the perketals as crosslinking and vulcanization agents. In these patents the peroxygen groups are bonded directly to the piperidine ring of the HALS. The fragmentation of perketals during the initiation process assures that this particular combination of HALS and perketal initiator would not effectively bond the HALS to the polymer. (S. W. Bukata, L. L. Zabrocki, I&EC Product Research and Development, 1964(3), pp 261-264; V. V. Zaitseva, A. I. Yurzhenko, J. Org. Chem. USSR, 4(8), pp 1350-1353 (1968)). A perketal in which the peroxygen groups are not bonded directly to the piperidine ring would leave the piperidine stabilizer intact after the initiation process. Such remote perketal groups are envisioned as part of the current invention.

The present invention is directed to novel compounds which contain both a free radical initiator and a hindered amine light stabilizer.

The compound of this invention is a free radical initiator containing a hindered amine light stabilizer group having the formula:

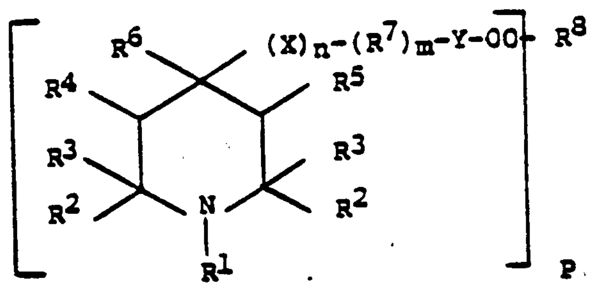

wherein:

p is 1 or 2.

n and m are independently selected from 0 and 1 with the proviso that when m is 0, n is 0.

$R^1$ is selected from hydrogen, alkyl of 1 to 8 carbons, alkenyl of 2 to 8 carbons, alkynyl of 2 to 8 carbons, cycloalkyl of 5 to 6 carbons in the ring, or aralkyl of 7 to 15 carbons. Preferably, $R^1$ is hydrogen, alkyl of 1 to 4 carbons, aralkyl of 7 to 9 carbons, or cycloalkyl of 5 to 6 carbons. Most preferably, $R^1$ is hydrogen, or alkyl of 1 to 4 carbons.

$R^2$ and $R^3$ may be the same or different and are independently selected from alkyl or 1 to 12 carbons, aryl of 6 to 10 carbons, aralkyl of 7 to 15 carbons, and $R^2$ and $R^3$ can be taken together with the carbon to which they are attached forming a saturated alicyclic group of 4 to 10 carbons, optionally containing oxygen or nitrogen in the ring. Preferably, $R^2$ and $R^3$ are selected from alkyl of 1 to 3 carbons, aralkyl of 7 to 10 carbons, and taken together with the carbon to which they are attached forming a saturated alicyclic ring of 6 carbons. Most preferably, $R^2$ and $R^3$ are selected from methyl, ethyl, or benzyl.

$R^4$ and $R^5$ may be the same or different and are independently selected from hydrogen, alkyl of 1 to 8 carbons, aryl of 6 to 10 carbons, aralkyl of 7 to 15 carbons. Preferably, $R^4$ and $R^5$ are selected from hydrogen, methyl, ethyl, or phenyl.

$R^6$ is selected from hydrogen, alkyl of 1 to 8 carbons, alkenyl of 2 to 8 carbons, alkynyl of 2 to 8 carbons, cycloalkyl of 5 to 6 carbons, alkoxy of 1 to 8 carbons, alkoxycarbonyl of 2 to 7 carbons, aryl of 6 to 10 carbons, aralkyl of 7 to 15 carbons, acyl of 1 to 8 carbons, aroyl of 7 to 16 carbons, alkylsulfonyl of 1 to 8 carbons, arylsulfonyl of 6 to 10 carbons, acyloxy of 1 to 7 carbons, aroyloxy of 6 to 10 carbons; $R^6$ may be joined to $R^7$ forming a cyclic structure of 5 to 7 atoms. Preferably, $R^6$ is hydrogen, alkyl of 1 to 4 carbons, alkoxycarbonyl of 2 to 5 carbons, acyloxy of 1 to 4 carbons, aryl of 6 carbons, or alkoxy of 1 to 3 carbons, Most preferably, $R^6$ is hydrogen, alkyl of 1 to 4 carbons, or alkoxy of 1 to 3 carbons.

X is selected from -O-, -S-, -N($R^9$)-, -C(=O)-,-S(=O)-, -S(=O)$_2$-, -O-C(=O)-, -O-S(=O)-,-O-S(=O)$_2$-, -N($R^9$)-C(=O)-, -NH-C(=O)-NH-, or -O-C(=O)-O-. Preferably, X is -O-, -S-, -N($R^9$)-, -O-C(=O)-O-, -N($R^9$)-C(=O)-O-, -O-C(=O)-, or -C(=O)-. Most preferably, X is -O-, -N($R^9$)-, -O-C(=O)-O-, -N($R^9$)-C(=O)-O-, or -O-C(=O)-.

$R^7$ is a diradical selected from alkylene of 1 to 20 carbons, arylene of 6 to 10 carbons, cycloalkylene of 3 to 10 carbons, aralkylene of 7 to 20 carbons, alkynylene of 2 to 10 carbons, alkadiynylene of 4 to 10 carbons, alkenylene of 3 to 11 carbons, alkadienylene of 5 to 11 carbons, optionally containing oxygen, sulfur, or nitrogen atoms in the diradical chain. Preferably, $R^7$ is selected from alkylene of 1 to 8 carbons, arylene of 6 to 10 carbons, aralkylene of 8 to 16 carbons, or cycloalkylene of 4 to 8 carbons. Most preferably, $R^7$ is alkylene of 1 to 6 carbons, arylene of 6 carbons; aralkylene of 9 to 12 carbons, or cycloalkylene of 5 to 7 carbons.

Y is selected from -C(=O), -S(=O)$_2$-, -C($R^{10}$)($R^{11}$)-, -O-C(=O)-, -N($R^9$)-C(=O)-, -O-C(=O)-C(=O)-. Preferably, Y is selected from -C(=O)-, -C($R^{10}$)($R^{11}$)-, or -O-C(=O)-.

When p is 1, $R^8$ is selected from hydrogen, acyl of 2 to 20 carbons, aroyl of 7 to 20 carbons, t-alkyl of 4 to 12 carbons, t-cycloalkyl of 4 to 12 carbons, t-aralkyl of 9 to 15 carbons, alkoxycarbonyl of 2 to 20 carbons, carbamoyl, phenylcarbamoyl, alkylcarbamoyl of 2 to 13 carbons, cycloalkylcarbamoyl of 4 to 13 carbons, alpha-hydroxyalkyl of 2 to 10 carbons, alpha-hydroxycycloalkyl of 3 to 10 carbons, alkylsulfonyl of 4 to 20 carbons, cycloalkylsulfonyl of 3 to 12 carbons, t-(alkoxyalkyl) of 4 to 20 carbons, t-(alkoxycycloalkyl) of 4 to 20 carbons, monovalent organomineral, or

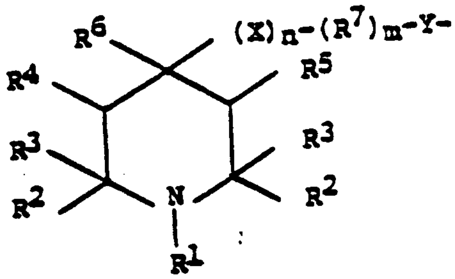

Preferably, $R^8$ is selected from acyl of 2 to 10 carbons, aroyl of 7 to 10 carbons, t-alkyl of 4 to 8 carbons, t-aralkyl of 9 to 16 carbons, or

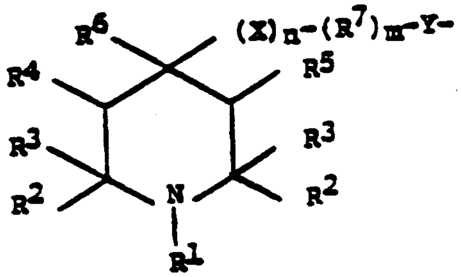

Most preferably, $R^8$ is selected from acyl of 2 to 10 carbons, aroyl of 7 carbons, t-alkyl of 4 to 6 carbons, t-aralkyl of 9 to 12 carbons, or

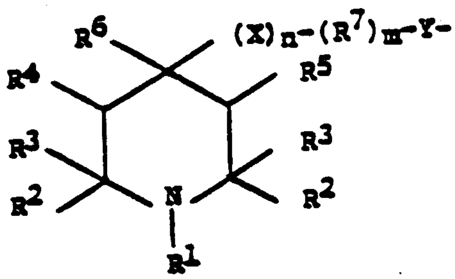

When p is 2, $R^8$ is a diradical selected from di-tertiary alkylene of 7 to 15 carbons, di-tertiary alkenylene of 8 to 16 carbons, di-tertiary alkynylene of 8 to 16 carbons, di-tertiary aralkylene of 12 to 20 carbons, alkanedioyl of 3 to 12 carbons, aryldicarbonyl of 8 to 16 carbons, or aralkyldicarbonyl of 9 to 18 carbons. Preferably, $R^8$ is selected from di-tertiary alkylene of 8 to 12 carbons, di-tertiary aralkylene of 12 to 15 carbons, or alkanedioyl of 3 to 6 carbons. Most preferably, $R^8$ is selected from di-tertiary alkylene of 8 to 10 carbons, di-tertiary aralkylene of 12 to 15 carbons, or alkanedioyl of 4 to 6 carbons.

$R^9$ is selected from hydrogen, alkyl of 1 to 8 carbons, alkenyl of 2 to 8 carbons, alkynyl of 2 to 8 carbons, cycloalkyl of 5 to 6 carbons in the ring, aryl of 6 to 10 carbons, acyl of 1 to 8 carbons; $R^9$ may be joined to $R^7$ forming a cyclic structure of 5 to 7 atoms. Preferably, $R^9$ is selected from hydrogen, alkyl of 1 to 4 carbons, cycloalkyl of 6 carbons, or aryl of 6 carbons. Most preferably, $R^9$ is hydrogen, or alkyl of 1 to 4 carbons.

$R^{10}$ is selected from alkyl of 1 to 10 carbons, aryl of 6 to 10 carbons, alkynyl of 2 to 10 carbons, alkenyl of 2 to 8 carbons, or cycloalkyl of 5 to 6 carbons in the ring. Preferably, $R^{10}$ is alkyl of 1 to 6 carbons, aryl of 6 to 10 carbons, or cycloalkyl of 5 to 6 carbons. Most preferably, $R^{10}$ is alkyl of 1 to 4 carbons, aryl of 6 carbons, or cycloalkyl of 6 carbons.

$R^{11}$ is selected from the same definition as for $R^{10}$; $R^{10}$ and $R^{11}$ can be connected to each other by means of an alkylene diradical bridge containing 4 to 9 carbons; when $R^8$ is t-alkyl, t-cycloalkyl, or t-aralkyl, $R^{11}$ may also be selected from -OO-$R^8$.

Optional substituents for $R^1$, $R^2$, $R^3$, $R^4$, $R^5$, $R^6$, $R^7$, $R^8$, $R^9$, $R^{10}$, and $R^{11}$ include halogen (-Cl, -Br), lower alkyl of 1 to 4 carbons, lower alkoxy of 1 to 4 carbons, -C≡N, -OH, epoxy

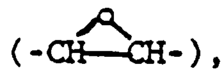

carboxy, alkyoxycarbonyl of 2 to 6 carbons, acyloxy of 1 to 4 carbons, hydroxymethyl, hydroxyethyl, and alkylmercapto of 1 to 4 carbons.

Some examples of compounds according to the present invention are as follows:

1) 00-t-butyl 0-(1,2,2,6,6-pentamethyl-4-piperidinyl) monoperoxycarbonate;
2) 1,3-dimethyl-3-t-butylperoxybutyl 1,2,2,6,6-pentamethyl-4-piperidinyl carbonate;
3) 00-t-butyl 0-(2,2,6,6-tetramethyl-4-piperidinyl) monoperoxycarbonate;
4) t-butyl 4-(1,2,2,6,6-pentamethyl-4-piperidinyloxy)-4-(oxo)peroxybutanoate;
5) 0-(1,3-dimethyl-3-t-butylperoxybutyl) N-(2,2,6,6-tetramethyl-4-piperidinyl) carbamate;
6) t-butyl 4-(2,2,6,6-tetramethyl-4-piperidinylamino)-4-(oxo)peroxybutanoate;
7) 00-t-butyl N-methyl-N-(2,2,6,6-tetramethyl-4-piperidinyl) peroxycarbamate;
8) di-(4-(2,3,6-trimethyl-2,6-diethyl-4-piperidinyloxy)-4-oxobutanoyl) peroxide;
9) 2,5-dimethylhexane-2,5-diyl bis (2-(1,2,2,6,6-pentamethyl-4-piperidinyloxy)peroxyacetate);
10) cyclohexylsulfonyl 2-(2,2,6,6-tetramethyl-4-piperidinyloxy)acetyl peroxide;
11) 00,00′-(2,5-dimethylhexane-2,5-diyl) bis(0-(2,2,6,6-tetramethyl-4-piperidinyl) monoperoxycarbonate);
12) di(1,1-dimethyl-3-(N-methyl-N-(1-ethyl-2,2,6,6-tetramethyl-4-piperidinyl)aminocarbonyloxy)butyl) peroxide;
13) 1-methyl-1-phenylethyl 6-(1,2,2,6,6-pentamethyl-4-piperidinyloxy)-6-oxo-4-(thia)peroxyhexanoate;
14) 1,1,3,3-tetramethylbutyl 5-(7-aza-15-methyldispiro(5.1.5.3)-15-hexadecyloxy)-5-oxo-3-(oxa)-peroxypentanoate;
15) 0-(1,3-dimethyl-3-(1,1-dimethylpropylperoxy)butyl) N-benzyl-N-(2,2,6,6-tetramethyl-4-piperidinyl) carbamate;
16) 00-t-butyl 0-(2-(1-(2,3-epoxypropyl)-2,2,6,6-tetramethyl-4-piperidinyloxycarbonyl)oxyethyl) monoperoxycarbonate;
17) 00-t-butyl 0-(1,2,2,6,6-pentamethyl-4-piperidinyl) monperoxyphthalate;
18) 1,1′-(1,4-phenylene)-bis(1-methylethyl) bis(2-(N-methyl-N-(2,2,6,6-tetramethyl-4-piperidinyl)amino)-peroxyacetate);
19) 1-methylcyclohexyl 3-(N-(1-(2-propenyl)-2,2,6,6-tetraethyl-3,5-dimethyl-4-piperidinyl)-N-phenylamino)-peroxypropanoate;
20) t-butyl 3-methyl-5-(1-benzyl-2,2,6,6-tetramethyl-4-piperidinyloxy)-5-oxo-3-(aza)peroxypentanoate;
21) 00-t-butyl 0-(1-(n-octyl)-2,2,6,6-pentamethyl-4-piperidinyl) monoperoxyoxalate;
22) t-butyl 4-(N-methyl-N-(1-(2-chloroethyl)-2,2,6,6-tetramethyl-4-piperidinyl)aminosulfonyl)-peroxybenzoate;
23) 4-(t-butylperoxycarbonyl)-N-(2,2,6,6-tetramethyl-4-piperidinyl)phthalimide;
24) t-butyl 2-(N-methyl-N-(2,2,6,6-tetramethyl-4-piperidinyl)amino)peroxyacetate;
25) 00-t-butyl 0-(7,9-dimethyl-8,8,10,10-tetramethyl-9-aza-1,5-dioxaspiro(5.5)undecane-3-yl) monoperoxycarbonate;
26) t-butyl 3-carboxy-4-(2,2,6,6-tetramethyl-4-piperidinyloxycarbonyl)peroxybenzoate;
27) 1,2,2,6,6-pentamethyl-4-piperidinyl 3,3-di-(t-butylperoxy)butanoate;
28) t-amyl (1,2,2,6,6-hexamethyl-4-piperidinyl)-peroxycarboxylate;
29) 1-hydroxy-3,3,5-trimethylcyclohexyl 2-(1-ethoxycarbonylmethyl-2,2,6,6-tetramethyl-4-piperidinyloxy)-peroxyacetate;
30) 1,1,3,3-tetramethyl 2,5-dimethyl-5-(2,2,6,6-tetramethyl-4-methoxy-4-piperidinylcarbonyloxy)-3-hexyne-2-yl peroxide.
31) 1,2,2,6,6-pentamethyl-4-piperidinyl 3,3-di(t-butylperoxy)-butanoate.

The novel HALS-peroxide initiators of this invention can be prepared by a variety of techniques which are well-known in the art. Two preferred methods include: a) reaction of HALS having pendant OH, SH or NH groups with peroxides containing acylating or alkylating functions, b) reaction of HALS containing acylating or alkylating functions with peroxides containing reactive OH, SH, NH, OOH or carboxy groups, or c) reaction of HALS containing ketone carbonyl with sufficient hydroperoxide to form the diperoxyketal. Examples of acylating groups include (but are not limited to) acid chlorides, chloroformates and isocyanates. Examples of alkylating groups are, for instance, reactive halogen or epoxide-containing compounds. The reaction to form the HALS-peroxide initiators can be done using any conditions which promote the reaction to create the desired product, and include the use of solvent (ethers, aromatic and nonaromatic hydrocarbons, chlorinated hydrocarbons, among others), acids and bases as may be required

to facilitate the formation. The application of heat may also be necessary for reaction to occur. However, the use of heat is possible only in so far as to accomplish the desired reaction without causing thermal decomposition of the peroxide group (the thermal or catalytic decomposition of this group is required in the subsequent use of the invention to prepare polymer and cure unsaturated polyester resin).

Unsaturated polyester resins that can be cured by the composition of this invention usually include an unsaturated polyester and one or more polymerizable monomers. The unsaturated polyesters are, for instance, obtained by esterifying at least one ethylenically unsaturated di- or polycarboxylic acid, anhydride, or acid halide, such as maleic acid, fumaric acid, glutaconic acid, itaconic acid, mesaconic acid, citraconic acid, allylmalonic acid, allylsuccinic acid, tetrahydrophthalic acid and others with saturated or unsaturated di- or polyols, such as ethylene glycol, diethylene glycol, triethylene glycol, 1,2- and 1,3-propanediols, 1,2-, 1,3- and 1,4-butanediols, 2,2-dimethyl-1,3-propanediols, 2,2-dimethyl-1,3-propanediol, 2-hydroxymethyl-2-methyl-1,3-propanediol, 2-buten-1,4-diol, 2-butyn-1,4-diol, 2,2,4-trimethyl-1,3-pentanediol, glycerol, pentaerythritol, mannitol and others. Mixtures of such polyacids and/or mixtures of such polyalcohols may also be used. The unsaturated di- or polycarboxylic acids may be partially replaced by saturated polycarboxylic acids, such as adipic acid, succinic acid, sebacic acid, and others and/or by aromatic polycarboxylic acids, such as phthalic acid, trimellitic acid, pyromellitic acid, isophthalic acid, and terephthalic acid. The acids used may be substituted by groups such as halogen. Examples of such suitable halogenated acids are, for instance, tetrachlorophthalic acid, 5,6-dicarboxy-1,2,3,4,7,7-hexachlorobicyclo(2.2.1)heptene, and others.

The other component of the unsaturated polyester resin, the polymerizable monomer or monomers, are preferably ethylenically unsaturated monomers, such as styrene, chlorostyrene, vinyltoluene, divinylbenzene, alpha-methylstyrene, diallyl maleate, diallyl phthalate, dibutyl fumarate, acrylonitrile, triallyl phosphate, triallyl cyanurate, methyl acrylate, methyl methacrylate, n-butyl methacrylate, ethyl acrylate, and others or mixtures thereof, which are copolymerizable with said polyesters.

A preferred unsaturated polyester resin contains as the polyester component the esterification product of 1,2-propylene glycol (a polyalcohol), maleic anhydride (an anhydride of an unsaturated polycarboxylic acid) and phthalic anhydride (an anhydride of an aromatic dicarboxylic acid) as well as the monomer component, styrene.

Other unsaturated polyester resins that are useful in the practice of this invention are unsaturated vinyl ester resins, consisting of a vinyl ester resin component and one or more polymerizable monomer components. The vinyl ester resin component can be made by reacting a chloroepoxide such as epichlorohydrin with appropriate amounts of a glycol such as bisphenol A (2,2-di-(4-hydroxyphenyl)-propane), in the presence of a base such as sodium hydroxide, to yield a condensation product having terminal epoxy groups derived from the epichlorohydrin. Subsequent reaction of the condensation product with polymerizable unsaturated carboxylic acids in the presence or absence of acidic or basic catalysts, results in the formation of a vinyl ester terminated resin component. Normally, styrene is added as the polymerizable monomer component to complete the preparation of the unsaturated vinyl ester resin.

Temperatures of 20 to 200°C and peroxide levels of 0.05 to 5% or more by weight of curable unsaturated polyester resin are normally employed in the curing process. The unsaturated polyester resins described above can be filled with various materials such as sulfur, glass fibers, carbon blacks, silicas, metal silicates, clays, metal carbonates, antioxidants, heat and light stabilizers, sensitizers, dyes, pigments, accelerators, metal oxides such as zinc oxide, and blowing agents.

The hindered amine-peroxide compound of the present invention is useful as a free radical initiator system for the polymerization or copolymerization of an ethylenically unsaturated monomer or mixtures thereof at suitable temperatures and pressures. The compound is useful not only in conventional isothermal polymerization processes but also in processes in which two or more increasing temperature steps are employed or a continuous increase in temperature is employed. Ethylenically unsaturated monomers include: olefins such as ethylene, propylene, styrene, alpha-methyl styrene, chlorostyrene, vinyl benzyl chloride, vinyl toluene, vinyl pyridine, divinyl benzene; diolefins such as 1,3-butadiene, isoprene and chloroprene; vinyl esters such as vinyl acetate, vinyl propionate, vinyl laurate, vinyl benzoate or divinyl carbonate; unsaturated nitriles such as acrylonitrile and methacrylonitrile; acrylic acid, methacrylic acid and their esters and amides, such as methyl ethyl, n-butyl and 2-ethylhexyl acrylates and methacrylates and acrylamide and methacrylamide; maleic anhydride; maleimide and N-substituted derivatives thereof such as n-phenylmaleimide; maleic and fumaric acids and their esters; vinyl halo and vinylidene halo compounds such as vinyl chloride, vinyl bromide, vinyl fluoride, vinylidene chloride and vinylidene fluoride; perhalo olefins such as tetrafluoroethylene, hexafluoropropylene and chlorotrifluoroethylene; vinyl ethers such as methyl vinyl ether, ethyl vinyl ether, n-butyl vinyl ether; allyl esters such as allyl acetate, allyl benzoate, diallyl phthalate, allyl ethyl carbonate, triallyl phosphate, triallyl cyanurate, diallyl fumarate, diallyl succinate, and diallyl carbonate; acrolein; methyl vinyl ketone; and mixtures thereof.

Temperatures of 30° to 250°C, preferably 40° to 200°C, and peroxide levels of 0.005 to 3%, preferably 0.01 to 1%, by weight, based on monomer, are normally employed in the conventional polymerization or in the increasing temperature polymerization processes. Polymerization can be carried out in solution where solvents such as toluene may be used. Bulk, solution, suspension, or emulsion polymerization processes may be employed. The hindered amine-peroxide composition of this invention may be employed in these vinyl polymerization processes alone or together with other peroxides and azo initiators.

The hindered amine-peroxide composition of this invention is also useful for producing high impact polymers such as high impact polystyrene by initiating grafting of a monomer onto the backbone of elastomers (rubbers) such as polybutadienes, styrene-butadiene-styrene triblock copolymers, and ethylene-propylene-diene terpolymers. This composition is also useful with lower amounts of the rubber to produce high impact resistant polymers having impact resistance comparable to high impact polymers produced with larger amounts of rubber and conventional initiator systems. The above described vinyl polymerization conditions and initiator levels and up to 15% by weight of rubber (based on monomer) may be used for producing high impact polymers.

In the following examples, polymer molecular weight, when given, was determined by standardized gel permeation chromatography (GPC) based on narrow molecular weight distribution polystyrene calibration. The reported molecular weight data include Mn, Mw, and Mz (W. W. Yau, J. J. Kirkland, Modern Size-Exclusion Liquid Chromotography, John Wiley & Sons (New York), 1979, pp 4-14). Precision of the experimental determination is ±5% for Mw. Mn and Mz are slightly less precise, ±5 to 10%. The molecular weights were determined on a Waters Associates ALC-GPC 244 with Model 6000A solvent delivery system and Model R401 differential refractometer as detector. The four columns used were Waters Ultra-Styragel™ with nominal pore sizes of $10^5$ nm, $10^4$ nm, $10^3$ nm and $10^2$ nm ($10^6$ Å, $10^5$ Å, $10^4$ Å, and $10^3$Å). The solvent was tetrahydrofuran, polymer samples for analysis were 0.2 to 0.3% by weight in the same solvent. Calibration for this system was done using TSK narrow molecular weight distribution polystyrene standards (range 500-$10^6$ Mw). Data handling was done using a Varian Model 401 chromatography data system interfaced with an Apple II+ ™ computer with Varian GPC software.

## EXAMPLE I

Preparation of 00-t-butyl 0-(1,2,2,6,6-pentamethylpiperidin-4-yl) monoperoxycarbonate (C-I)

1. Preparation of 1,2,2,6,6-pentamethyl-4-(chlorocarbonyloxy)piperidinium chloride.

Into a 2 L round bottom flask equipped with an oil bath, reflux condenser, and nitrogen atmosphere sparger were charged 1,2,2,6,6-pentamethyl-4-piperidinol (51.2g, 0.3 mol) and 1 L of 2-butanone. Freshly distilled phosgene (35 ml, 0.45 mol) was evaporated into the reactor in a stream of nitrogen; a solid precipitate formed immediately. The resulting slurry was heated to reflux which initially was 53°C but rose gradually over 25 minutes to 77°C during which time the initially formed solid dissolved. After an additional 25 minutes of reflux at 77°C, the reaction mixture was cooled while being purged of excess phosgene and hydrogen chloride with a nitrogen stream. The ketone solvent was stripped from the product using an aspirator vacuum. The solid obtained was isolated and washed with 300 ml of 1,1,1-trichloroethane; then it was washed a second time with 200 ml of 1,1,1-trichloroethane. The resulting white crystalline solid was dried under high vacuum to give 76.5g of the chloroformate hydrochloride having an assay of 99.7% (by analysis of hydrolyzable chloride: theoretical 26.24%, found 26.17%) and thus a corrected yield of 94.4%.

2. Preparation of C-I.

Into a 1 L flask equipped with an oil bath, thermometer, mechanical stirrer, reflux condenser, and nitrogen atmosphere sparger were combined poly-4-vinylpyridine (105.8g, 0.9 equivalents) and 300 ml of toluene. The mixture was heated and 50 ml of toluene were collected (removed air and water from polymer). The mixture was cooled to ambient temperature and the reflux condenser was replaced with an addition funnel for the addition of t-butyl hydroperoxide (206.8g, 2.2 mol), which was accompanied by a slight exotherm. After adjusting the temperature to 20°C using an external ice/water bath, 1,2,2,6,6-pentamethyl-4-(chlorocarbonyloxy)piperidinium chloride (37.9g, 0.14 mol) was added, again using an ice/water bath to control the slight exotherm and maintain the reaction mixture temperature 20-25°C. The reaction mixture thus formed was allowed to stir at ambient temperature for four hours. The mixture was cooled and the poly-4-vinylpyridine was filtered from the solution with the aid of a filter pad of anhydrous sodium sulfate. The

polymer and sulfate were rinsed with 3ℓ of pentane. The combined organic solutions were stripped of solvent using an aspirator vacuum and the residue was transferred to a separatory funnel along with 450 ml of pentane. The organic solution was washed four times with 150 ml portions of 5% aqueous sodium hydroxide then dried using anhydrous magnesium sulfate. The desiccant was filtered from the solution and the solution was treated with methylene chloride which had been saturated with hydrogen chloride. This caused the formation of a white crystalline solid. The solid was isolated and transferred to a separatory funnel with 200 ml of methyl t-butyl ether and 200 ml of 5% aqueous sodium hydroxide. The phases were separated and the organic solution was dried with anhydrous magnesium sulfate. The desiccant was filtered from the solution and the solvent was removed using aspirator vacuum. The desired monoperoxycarbonate was obtained as a slightly yellow liquid weighing 27.7g. Analysis indicated an assay of 93.7% and therefore a corrected yield of 64.6%. Further structural proof was obtained from a proton NMR spectrum which agreed with the expected structure.

## EXAMPLE II

### Preparation of 1,3-dimethyl-3-t-butylperoxybutyl 1,2,2,6,6-pentamethyl-4-piperidyl carbonate (C-II)

1. Preparation of 1,2,2,6,6-pentamethyl-4-piperidinol, sodium salt.

Into a dried 250 ml. flask equipped with a reflux condenser and an oil bath were placed 7.45g (.0435 mol) of 1,2,2,6,6-pentamethyl-4-piperidinol, 1.35g (.0587 mol) of sodium, and 100 ml of xylene; the mixture was heated to reflux for 16 hours and then cooled; and the unreacted sodium (.225g) was removed from the mixture. The alkoxide solution was used in the next step.

2. Preparation of C-II

Into a dried 500 ml flask equipped with magnetic stirbar, addition funnel, nitrogen atmosphere sparger, and a Dry Ice/acetone cooling bath were placed 11.6g (.0435 mol) of 3-t-butylperoxy-1,3-dimethylbutyl chloroformate and 30 ml of acetonitrile. The alkoxide prepared above was charged into the addition funnel with 20 ml of acetonitrile. The reaction mixture was cooled to less than 5°C and the alkoxide was added dropwise over a 20 minute period forming an immediate precipitate and generating considerable foam. After the addition was completed, the reaction mixture was stirred for 1 hour at 0-5°C and then for 2.5 hours at ambient temperature. The reaction mixture was transferred to a separatory funnel with 100 ml of methyl t-butyl ether and extracted with three 50 ml portions of 5% sodium hydroxide and three 50 ml portions of water. The organic phase was dried with anydrous magnesium sulfate and the solvent was stripped using an aspirator vacuum to remove the acetonitrile and leaving the xylene. A methylene chloride solution saturated with hydrogen chloride was added dropwise to the xylene solution until the solution was acidic to moist pH paper. The hydrochloride salt of the C-II compound was precipitated by the addition of 500 ml of pentane. The salt was filtered and transferred to a separatory funnel containing 100 ml of methyl t-butyl ether and 100 ml of 5% sodium hydroxide. The mixture was shaken and the phases were allowed to separate. The aqueous phase was removed and the organic solution was washed once with 50 ml of 5% sodium hydroxide and once with 50 ml of water. The organic mixture was dried using anhydrous magnesium sulfate and then stripped of solvent using an aspirator vacuum. The liquid residue was taken up in 50 ml pentane which caused some unreacted piperidinol to precipitate. This was removed by filtration and the pentane solution was washed twice with very dilute aqueous acetic acid solution (1 drop conc. acetic acid/100 ml water) and once with 50 ml 5% sodium hydroxide. The pentane solution was dried again with anhydrous magnesium sulfate and the solvent stripped using an aspirator vacuum. The desired peroxide was thus obtained as a slightly yellow liquid weighing 5.2g. Liquid chromatographic analysis indicated that the product consisted essentially of one component. Assuming 100% assay, the corrected yield was 30.8%. The NMR spectrum of the product confirmed the structure for C-II.

When 2,2,6,6-tetramethyl-4-piperidylamine and a tertiary amine base are substituted for the piperidyl alkoxide used in this preparation, reaction with 3-t-butylperoxy-1,3-dimethylbutylchloroformate produces 0-(1,3-dimethyl-3-t-butylperoxybutyl) N-(2,2,6,6-tetramethyl-4-piperidyl) carbamate.

## EXAMPLE III

### Preparation of 00-t-butyl 0-(2,2,6,6-tetramethyl-4-piperidinyl) monoperoxycarbonate (C-III)

1. Preparation of 4-chlorocarbonyloxy-2,2,6,6-tetramethylpiperidinium chloride

Into a 500 ml flask equipped with a magnetic stirbar, thermometer, gas inlet adapter, dual condensers (i.e., Dry Ice condenser atop a water cooled condenser), and oil bath were placed 28.2g (.145 mol) of 2,2,6,6-tetramethyl-4-piperidinol hydrochloride and 250 ml of 2-butanone. The mixture was heated to reflux and 8.0 ml (0.11 mol) of condensed phosgene was allowed to evaporate into the reaction mixture cooling the mass to 73-75°C. This was followed by pipet addition of 11.5g. (.145 mol) of pyridine which caused an exothermic reaction raising the temperature up to 80°C. An additional 10 ml (0.14 mol) of phosgene was evaporated into the system. During this time the reaction mixture cleared of initially suspended solid and then formed a precipitate. After the addition of phosgene was completed, nitrogen was bubbled into the system for 40 minutes under continued reflux. The mixture was then allowed to cool gradually overnight and then was cooled further in an ice water bath. The very hygroscopic solid was collected by filtration. The liquid filtrate was reduced to 50 ml volume by distillation at atmospheric pressure, cooled to ambient, and diluted with 100 ml of tetrahydrofuran. In this manner additional solids were obtained and isolated by filtration. The combined solids were then freed from residual solvent under high vacuum. The product was a mixture of the tetramethylpiperidium chloride compound and pyridinium hydrochloride. This mixture was used in the next step without further purification.

2. Preparation of C-III

Into a 500 ml flask equipped with a magnetic stirbar, thermometer, and Dry Ice/acetone cooling bath were placed 204.2g of aqueous 15% potassium hydroxide (.546 mol KOH). This was cooled to -5° to 0°C and 51.9g (.52 mol) t-butyl hydroperoxide (assay 90.3%) were added by pipe maintaining the aforementioned temperature range. The solution was transferred to a separatory funnel, was extracted with 50 ml of hexane, was then returned to the reaction vessel, and was again cooled to -5°C. Tetrahydrofuran (45 ml) was added forming an upper layer. The salt mixture (prepared in 1. above) was added in small portions, using a cooling bath to maintain -5°C. The reaction mass thickened and 25 ml methyl t-butyl ether was added to facilitate stirring. The reaction mixture was allowed to warm to ambient temperature and stirred for 3 hours. The mixture was transferred to a separatory funnel with 250 ml of methyl t-butyl ether and the phases were allowed to separate. The aqueous phase was removed and the organic phase was extracted with two 50 ml portions of 15% potassium hydroxide, two 50 ml portions of cold water, and 50 ml of buffered sulfite solution (prepared from water, acetic acid, sodium acetate, and sodium sulfite). Before removal of the buffered sulfite wash, it was made basic by addition of 15% potassium hydroxide. A final wash with 50 ml of 5% sodium bicarbonate was performed. The organic solution was stripped of solvent using an aspirator vacuum. The residue was mixed with 30 ml water and the pyridine/water azeotrope was removed under high vacuum. The semi-solid aqueous residue was dissolved in 120 ml methyl t-butyl ether and transferred to a separatory funnel. Solid sodium chloride was added to prepare a saturated aqueous phase which was separated. The organic phase was dried using anhydrous magnesium sulfate and the solvent was stripped using aspirator and high vacuum systems. The yellow liquid residue solidified upon standing in the freezer. The solids were further high vacuum stripped for 4 hours, broken up with a mortar and pestle, then stripped again for 1.5 hours. The product was obtained as a white solid weighing 32.2g and having a melting point of 52-57°C. This product was identified as the 1:1 complex of the C-III compound and t-butyl hydroperoxide.

Into a 300 ml flask equipped with a magnetic stirbar, thermometer, addition funnel, and ice water cooling bath were placed 23.3g (0.064 mol) of the complex prepared above and 50 ml of methyl t-butyl ether. The addition funnel was charged with 93.1g of buffered sulfite solution and this solution was added dropwise to the complex mixture while maintaining the reaction temperature below 10°C. After the addition was completed, the cooling bath was removed the the solution was allowed to stir at ambient temperature for 1.5 hours. To the reaction mixture was then added 24.4g of aqueous 15% potassium hydroxide along with 25 ml of methyl t-butyl ether. The reaction mass was transferred to a separatory funnel. The aqueous phase was separated and extracted once with 20 ml of methyl t-butyl ether. The combined organic solutions were dried with anhydrous magnesium sulfate and stripped of solvent using aspirator and high vacuum systems. The residue crystallized upon cooling. The solids were dissolved in pentane and chilled to precipitate 2,2,6,6-tetramethyl-4-piperidinol. This impurity was removed by filtration and the liquid filtrate stripped using aspirator and high vacuum systems to give the C-III compound as a viscous yellow liquid weighing 12.4g. Analysis indicated an assay of 93.7% and a corrected yield of 29.2%.

EXAMPLE IV

Preparation of t-butyl 4-(1,2,2,6,6-pentamethyl-4-piperidinyloxy)-4-oxoperoxybutanoate (C-IV)

Into a 500 ml flask equipped with a magnetic stirbar, thermometer, addition funnel, and nitrogen atmosphere sparger were placed 7.6g (.044 mol) of 1,2,2,6,6-pentamethyl-4-piperidinol, 4.4g (.044 mol) of triethylamine, and 100 ml of pentane. The addition funnel was charged with 9.7g (.044 mol) of 4-t-butylperoxy-4-oxobutanoyl chloride, 50 ml of methyl t-butyl ether and 50 ml of pentane. The acid chloride was added dropwise to the reaction mixture at a rate so as to maintain the temperature of the reaction ≦27°C (about 70 min for complete addition). After this addition, the reaction mixture was allowed to stir at ambient temperature for 45 minutes. To the mixture 100 ml of aqueous 5% sodium hydroxide (chilled to 5°C) was then added with rapid stirring. The mixture was transferred to a separatory funnel with 100 ml of methyl t-butyl ether and 100 ml of pentane. The funnel was shaken and allowed to separate; and the aqueous phase was drawn off. The organic phase was extracted with 100 ml of aqueous 5% sodium hydroxide (chilled to 5°C), and twice with 100 ml portions of cold (5°C) water. The organic solution was dried with anhydrous sodium sulfate and the solvent was stripped using aspirator and high vacuum systems. The residue was transferred to a flask with 30 ml of pentane and treated with 5.3g of decolorizing carbon (occasional swirling for 90 minutes). The mixture was diluted with 200 ml of pentane, filtered free of carbon, and stripped of solvent using aspirator and high vacuum. The product was obtained as a yellow oil weighing 9.7g. Analysis indicated an assay of 96.1% and thus a corrected yield of 62.2%.

The use of 2,2,6,6-tetramethyl-4-aminopiperidine instead of 1,2,2,6,6-pentamethyl-4-piperidinol in this experiment produces t-butyl 4-(2,2,6,6-tetramethyl-4-piperidylamino)-4-(oxo)peroxybutanoate.

EXAMPLE V

Curing unsaturated polyester resin

The stabilizer-initiators of the instant invention were used to prepare polyester with bound stabilizer groups. Gelation and cure characteristics were determined using the Standard SPI Exotherm Procedure ("SPI Procedures for Running Exotherm Curves - Polyester Resin", published in the Preprint of the 16th Annual Conference - Reinforced Plastics Division, Society of the Plastics Industry, Inc., February 1961). The results are shown in Table I.

TABLE I

SPI Activity Comparison of Stabilizer-Peroxides

| Initiator | Gel Time (min) | Cure Time (min) | Exotherm Max °C (°F) | Barcol Hardness |
|---|---|---|---|---|
| C-I | 4.5 | 5.1 | 230 (446) | 35-45 |
| C-II | 7.0 | 8.0 | 222 (432) | 35-45 |
| C-III | 4.8 | 5.5 | 223 (433) | 35-45 |
| C-IV | 10.4 | 12.6 | 206 (402) | 35-45 |
| t-butyl perbenzoate | 5.1 | 5.8 | 214 (418) | 35-45 |
| di-t-butyl peroxide | 11.6 | 14.3 | 176 (348) | 30-40 |
| Di-cumyl peroxide | 6.5 | 7.8 | 203 (397) | 35-45 |
| Lupersol 233-MO90* | 6.0 | 6.7 | 221 (430) | 35-45 |

Initiator concentration: $4.0 \times 10^{-3}$ molar
Resin: E-4297-7 ( a one component low profile isophthalic molding resin marketed by Owens Corning Fiberglass)
Filler: 100 phr calcium carbonate
Cure Temperature: 127°C (260°F)
* Ethyl 3,3-di(t-butylperoxy)butyrate, 90% solution in paraffinic oil

EXAMPLE VI

Preparation of High Solids Coating Resin

Methyl n-amyl ketone (300g) was heated to 145°C in a jacketed glass reactor equipped with a stirrer, thermometer, reflux condenser, and nitrogen gas sparging line. A mixture consisting of 40g of methyl methacrylate, 53g of isobutyl methacrylate, 30g of 2-hydroxyethyl methacrylate, and 3.7 g of HALS peroxide C-IV was prepared. A 100g portion of this mixture was added uniformly at a rate of 25g per hour to the refluxing solvent over 4 hours. After the monomer/initiator addition was completed, the polymerization was continued for one hour. The coatings resin was obtained in 74% monomer conversion and had the following molecular weight data by GPC:

Mw = 11,000
Mn = 5,300
Mz = 18,000
Mw/Mn = 2.1

EXAMPLE VII

Preparation of poly(methylmethacrylate)

Methyl n-amyl ketone (300g) was heated to 120°C in a jacketed glass reactor equipped with a stirrer,

thermometer, reflux condenser and nitrogen gas sparging line. A mixture consisting of 150g of methyl methacrylate and 8.0g of HALS peroxide C-I was prepared. A 107g portion of this mixture was added uniformly at a rate of 26g per hour to the refluxing solvent over 4 hours. After monomer/initiator addition was completed, the polymerization was continued for two hours at reflux. The poly(methyl methacrylate) was obtained in 92% monomer conversion and had the following molecular weight data by GPC:

Mw = 5,580
Mn = 3,100
Mz = 8,400
Mw/Mn = 1.8

EXAMPLE VIII

Preparation of Polystyrene

The stabilizer-initiator C-III was used to polymerize styrene monomer using bulk polymerization conditions at 120 °C and 0.28 phm of initiator. The 1:1 complex of this initiator and t-butyl hydroperoxide was also used to polymerize styrene monomer under the same conditions using 0.25 phm of the complex. In each case, the polymerized mass was dissolved in toluene and the polymer precipitated by addition of the toluene solution to a large quantity of rapidly stirred methanol. The polystyrene produced was characterized by the data in Table II for percent conversion, viscosity average molecular weight, and Mn, Mw, and Mz (by GPC).

TABLE II

Bulk Polymerization of Styrene

Initiator: 00-t-butyl 0-(2,2,6,6-tetramethyl-4-piperidinyl monoperoxycarbonate (C-III)

| Reaction Time (min) | % conversion | Mv** | Mn | Mw | Mz |
|---|---|---|---|---|---|
| 30 | 36 | 99,000 | - | - | - |
| 50 | 56 | 111,000 | - | - | - |
| 90 | 89 | 153,000 | - | - | - |
| 100 | 99 | 163,000 | 89,000 | 230,000 | 461,000 |

TABLE II (continued)

Bulk Polymerization of Styrene

Initiator: OO-t-butyl O-(2,2,6,6-tetramethyl-4-piperidinyl) monoperoxycarbonate C-III, 1:1 complex with t-butyl hydroperoxide

| Reaction Time (min) | % conversion* | Mv** | Mn | Mw | Mz |
|---|---|---|---|---|---|
| 30 | 28 | 104,000 | - | - | - |
| 50 | 46 | 117,000 | - | - | - |
| 90 | 72 | 139,000 | - | - | - |
| 150 | 96 | 166,000 | - | - | - |

* - conversion was determined by precipitation of the polymer in methanol.
** - viscosity average molecular weight in toluene at 25°C.

EXAMPLE IX

Preparation of Poly(p-methylstyrene)

The stabilizer-initiator C-III was used to prepare a polymer from p-methylstyrene. The polymer was prepared in bulk using 0.28 phm of initiator and heating the reaction at 120°C for 150 minutes. The polymerized mass was dissolved in toluene and precipitation of the polymer was accomplished by dropwise addition of the toluene solution to rapidly stirred methanol. The polymer was isolated by filtration. The polymer prepared was analyzed by GPC with the following results:

Mw = 469,000
Mn = 105,000
Mz = 1,210,000
Mw/Mn = 4.5

EXAMPLE X

Accelerated Weather Testing

1. Sample Preparation

A stock solution (A) of 8% polybutadiene (TAKTENE XG 575, from Polysar) in styrene monomer was prepared; and .08 part (based on the weight of monomer blend) of initiator was added thereto. A second stock solution (B) of styrene containing .53 parts by weight of intiator was prepared. Solution A (62.5g) was placed in a reaction flask and heated in a 100°C oil bath for approximately 30 minutes until the solution had become distinctly turbid. The mass was cooled to 50°C and 37.5g of solution B was added. The resulting resin mixture was bulk polymerized at 120°C for 5 hours. The cured polymer was ground using a Brabender Granu-Grinder. The ground polymer was compression molded into approximately 0.71 - 0.81 mm (28-32 mil) thick specimens using a Carver hydraulic press, 160°C (320°F) and approx. 552 bar (8000 psi) for 2 minutes.

2. Test Procedure

Accelerated weathering was done in a QUV weathering tester made by Q Panel Company with a 8 hour 60°C light cycle and a 4 hour 50°C wet cycle. Samples were removed (in triplicate) at various time intervals and the molecular weight determined using GPC. The results are shown in TABLE III. The results demonstrate that the polymer prepared using the stabilizer initiator of this invention resisted degradation much better than polymer prepared using an initiator with no attached stabilizer.

TABLE III

Accelerated Weathering Test results

Initiator A was HALS peroxide C-I

Initiator B was Lupersol TBEC*

| | | Exposure Time (in hours) 0 | 500 | 1000 |
|---|---|---|---|---|
| % molecular weight retained using initiator A | Mn | 100 | 68.1 | 67.5 |
| | Mw | 100 | 79.8 | 76.6 |
| | Mz | 100 | 80.1 | 82.0 |
| % molecular weight retained using initiator B | Mn | 100 | 63.6 | 44.6 |
| | Mw | 100 | 73.3 | 62.3 |
| | Mz | 100 | 74.7 | 68.3 |

*OO-t-butyl O-(2-ethylhexyl) monoperoxycarbonate

EXAMPLE XI

Preparation of 4-(t-amylperoxycarbonyl)-N-(2,2,6,6-tetramethyl-4-piperidinyl)phthalimide (C-V)

1. Preparation of 4-carboxy-N-(2,2,6,6-tetramethyl-4-piperidinyl)phthalimide

Into a 125 ml flask equipped with thermometer, magnetic stirbar and condenser were combined (in order) acetic acid (250 ml), trimellitic anhydride (10.3g, .053 mol), potassium acetate (15.7g .16 mol) and 2,2,6,6-tetramethyl- 4-piperidinylamine (25.1g, .16 mol). The resulting mixture was heated to reflux. Most of the solids dissolved upon heating but were quickly replaced by a thick precipitate. The mixture was refluxed for 6 hours. The hot mixture was poured into a 1 L beaker packed with ice. The solution was stirred until the ice melted and the solid was isolated by filtration. The solid was slurried with 200 ml of tetrahydrofuran and filtered again. The solid was dried on the filter funnel. The product was 12.4g of white solid with melting point >225°C. Upon standing the original filtrate formed additional solids. These were isolated and combined with previously isolated product to give a total of 13.8g of material (78.4% of theoretical).

2. Preparation of 4-(chlorocarbonyl)-N-(2,2,6,6-tetramethyl-4-piperidinyl)phthalimide, hydrochloride salt

Into a 250 ml flask equipped with a magnetic stirbar and condenser were combined the acid prepared above (8.8g, .027 mol) and thionyl chloride (150 ml). The mixture was heated to reflux and refluxed for 1 hour (until cessation of gas evolution). The excess thionyl chloride was stripped using aspirator vacuum and the solid residue was isolated using methylene chloride. The solid was slurried with additional methylene

chloride and filtered a second time. The solid was dried briefly under high vacuum to give 10.3g of product (100% of theoretical).

3. Preparation of C-V

Into a 500 ml flask equipped with magnetic stirbar, thermometer and ice water bath were combined (in order) pyridine (100 ml), t-amyl hydroperoxide (14.4g, .13 mol, assay 97% by theoretical active oxygen) and the acid chloride hydrochloride prepared above (10.3g, .027 mol). All of the additions were done so as to maintain the reaction temperature 15-20 °C. None of the additions was noticeably exothermic. The mixture was warmed to ambient temperature and stirred for 45 minutes. The excess pyridine was stripped at ambient temperature using a high vacuum system. The viscous yellow oil which remained was transferred to a separatory funnel with 250 ml of methyl t-butyl ether and 100 ml of 5% sodium hydroxide. The mixture was shaken and the aqueous phase removed. The ether solution was washed with two more 100 ml portions of 5% sodium hydroxide, and three 100 ml portions of water. The organic solution was dried with anhydrous magnesium sulfate, the desiccant was filtered and the the solvent removed using aspirator and high vacuum systems. The solid residue was mixed with 100 ml tetrahydrofuran and 40 ml of buffered sulfite solution and stirred for 45 minutes. The mixture was transferred to a separatory funnel with 200 ml of methyl t-butyl ether and 100 ml of 5% sodium hydroxide. The mixture was shaken and the aqueous phase removed. The ether solution was washed with two 100 ml portions of water and one 50 ml portion of saturated aqueous sodium chloride. The organic phase was dried with anhydrous magnesium sulfate. The desiccant was filtered and the solvent stripped using aspirator and high vacuum systems. The residue was mixed with water and the water/pyridine azeotrope was distilled under high vacuum. The aqueous mixture was transferred to a separatory funnel and the product was extracted using methylene chloride. The methylene chloride extract was dried with anhydrous magnesium sulfate and the solvent stripped using aspirator and high vacuum systems. The yellow oil residue was mixed with a small amount of pentane which caused the product to crystallize. The product was isolated and dried on the funnel. The product purity was acertained by active oxygen analysis which indicated an assay of 92.2%. The melting range was 126-130 °C. The infrared spectrum further confirmed the product as the desired HALS-peroxide.

## Claims

**1.** A peroxide initiator containing hindered amine light stabilizer group(s) having the formula:

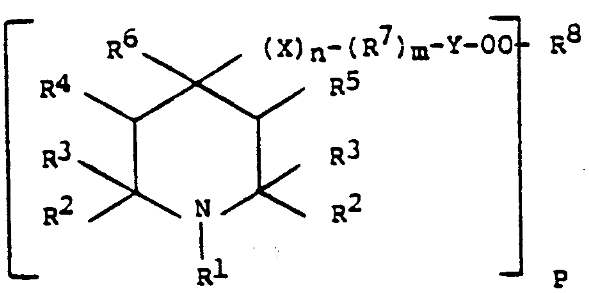

wherein:

p is 1 or 2,

n and m are independently selected from 0 and 1 with the proviso that when m is 0 then n is 0,

$R^1$ is selected from hydrogen, alkyl of 1 to 8 carbons, alkenyl of 2 to 8 carbons, alkynyl of 2 to 8 carbons, cycloalkyl of 5 to 6 carbons in the ring, aralkyl of 7 to 15 carbons,

$R^2$ and $R^3$ may be the same or different, and are independently selected from alkyl of 1 to 12 carbons, aryl of 6 to 10 carbons, aralkyl of 7 to 15 carbons and $R^2$ and $R^3$ can be taken together with the carbon to which they are attached forming a saturated alicyclic group of 4 to 10 carbons, optionally containing oxygen or nitrogen in the ring,

$R^4$ and $R^5$ may be the same or different and are independently selected from hydrogen, alkyl of 1 to 8 carbons, aryl of 6 to 10 carbons, aralkyl of 7 to 15 carbons,

$R^6$ is selected from hydrogen, alkyl of 1 to 8 carbons, alkenyl of 2 to 8 carbons, alkynyl of 2 to 8 carbons, cycloalkyl of 5 to 6 carbons, alkoxy of 1 to 8 carbons, alkoxycarbonyl of 2 to 7 carbons, aryl of

6 to 10 carbons, aralkyl of 7 to 15 carbons, acyl of 1 to 8 carbons, aroyl of 7 to 16 carbons, alkylsulfonyl of 1 to 8 carbons, arylsulfonyl of 6 to 10 carbons, acyloxy of 1 to 7 carbons, and aroyloxy of 6 to 10 carbons, and $R^4$ may be joined to $R^7$ forming a cyclic structure of 5 to 7 atoms,

X is selected from -O-, -S-, -N($R^9$)-, -C(=O)-, -S(=O)-, -S(=O)$_2$-, -O-C(=O)-, -O-S(=O)-, -O-S-(=O)$_2$-, -N($R^9$)-C(=O)-, -NH-C(=O)-NH-, -O-C(=O)-O-,

$R^7$ is a diradical selected from alkylene of 1 to 20 carbons, arylene of 6 to 10 carbons, cycloalkylene of 3 to 10 carbons, aralkylene of 7 to 20 carbons, alkynylene of 2 to 10 carbons, alkadiynylene of 4 to 10 carbons, alkenylene of 3 to 11 carbons, alkadienylene of 5 to 11 carbons, optionally containing oxygen, sulfur, or nitrogen atoms in the diradical chain,

Y is selected from -C(=O)-, -S(=O)$_2$-, -C($R^{10}$)($R^{11}$)-, -O-C(=O)- , -N($R^9$)-C(=O)- , -O-C(=O)-C-(=O)-,

when P is 1, $R^8$ is selected from hydrogen, acyl of 2 to 20 carbons, aroyl of 7 to 20 carbons, t-alkyl of 4 to 12 carbons, t-cycloalkyl of 4 to 12 carbons, t-aralkyl of 9 to 15 carbons, alkoxycarbonyl of 2 to 20 carbons, carbamoyl, phenylcarbamoyl, alkylcarbamoyl of 2 to 13 carbons, cycloalkylcarbamoyl of 4 to 13 carbons, alpha-hydroxyalkyl of 2 to 10 carbons, alpha-hydroxycycloalkyl of 3 to 10 carbons, alkylsulfonyl of 4 to 20 carbons, cycloalkylsulfonyl of 3 to 12 carbons, t-(alkoxyalkyl) of 4 to 20 carbons, t-(alkoxycycloalkyl) of 4 to 20 carbons, monovalent organomineral, or

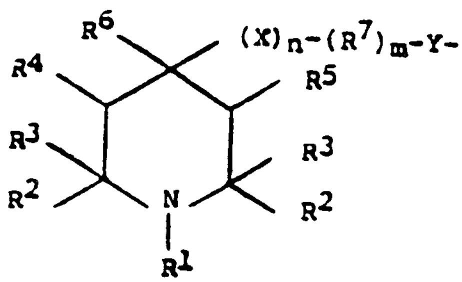

when p is 2, $R^8$ is a diradical selected from di-tertiary alkylene of 7 to 15 carbons, di-tertiary alkenylene of 8 to 16 carbons, di-tertiary alkynylene of 8 to 16 carbons, di-tertiary aralkylene of 12 to 20 carbons, alkanedioyl of 3 to 12 carbons, aryldicarbonyl of 8 to 16 carbons, or aralkyldicarbonyl of 9 to 18 carbons,

$R^9$ is selected from hydrogen, alkyl of 1 to 8 carbons, alkenyl of 2 to 8 carbons, alkynyl of 2 to 8 carbons, cycloalkyl of 5 to 6 carbons in the ring, aryl of 6 to 10 carbons, acyl of 1 to 8 carbons and $R^9$ may be joined to $R^7$ forming a cyclic structure of 5 to 7 atoms,

$R^{10}$ is selected from alkyl of 1 to 10 carbons, aryl of 6 to 10 carbons, alkynyl of 2 to 10 carbons, alkenyl of 2 to 8 carbons, cycloalkyl of 5 to 6 carbons in the ring,

$R^{11}$ is selected from the definition of $R^{10}$ and $R^{10}$ and $R^{11}$ can be connected to each other by means of an alkylene diradical bridge containing 4 to 9 carbons and when $R^8$ is t-alkyl, t-cycloalkyl or t-aralkyl, $R^{11}$ may be -OO-$R^8$,

substituents for $R^1$, $R^2$, $R^3$, $R^4$, $R^5$, $R^6$, $R^7$; $R^8$, $R^9$, $R^{10}$, and $R^{11}$ include halogen, alkyl of 1 to 4 carbons, alkoxy of 1 to 4 carbons, -C≡N, -OH, epoxy, carboxy, alkyoxycarbonyl of 2 to 6 carbons, acyloxy of 1 to 4 carbons, hydroxymethyl, hydroxyethyl, and-alkylmercapto of 1 to 4 carbons.

**2.** The compound of claim 1 in which $R^1$ Is selected from hydrogen, alkyl of 1 to 4 carbons, aralkyl of 7 to 9 carbons, or cycloalkyl of 5 to 6 carbons,

$R^2$ and $R^3$ may be the same or different, and are independently selected from alkyl of 1 to 3 carbons, aralkyl of 7 to 10 carbons, and can be taken together with the carbon to which they are attached forming a saturated alicyclic group of 6 carbons,

$R^6$ is selected from hydrogen, alkyl of 1 to 4 carbons, alkoxycarbonyl of 2 to 5 carbons, acyloxy of 1 to 4 carbons, aryl of 6 carbons, or alkoxy of 1 to 3 carbons,

X is -O-, -S-, -N($R^9$)-, -O-C(=O)-O-, -N($R^9$)-C(=O)-O-, -O-C(=O)-, -C(=O)-,

$R^7$ is selected from alkylene of 1 to 8 carbons, arylene of 6 to 10 carbons, aralkylene of 8 to 16 carbons, or cycloalkylene of 4 to 8 carbons,

when p is 1, $R^8$ is selected from acyl of 2 to 10 carbons, aroyl of 7 to 10 carbons, t-alkyl of 4 to 8 carbons, t-aralkyl of 9 to 16 carbons, or

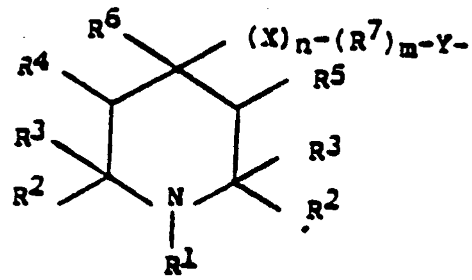

when p is 2, $R^8$ is selected from di-tertiary alkylene of 8 to 12 carbons, di-tertiary aralkylene of 12 to 15 carbons, or alkanedioyl of 3 to 6 carbons,

$R^9$ is selected from hydrogen, alkyl of 1 to 4 carbons, cycloalkyl of 6 carbons, aryl of 6 carbons, and

$R^{10}$ is alkyl of 1 to 6 carbons, aryl of 6 to 10 carbons, or cycloalkyl of 5 to 6 carbons.

**3.** The compound of claim 2 in which $R^1$ is selected from hydrogen or alkyl of 1 to 4 carbons,

$R^2$ and $R^3$ may be the same or different and are selected from methyl, ethyl, or benzyl,

$R^4$ and $R^5$ are the same or different and are selected from hydrogen, methyl, ethyl, or phenyl,

$R^6$ is selected from hydrogen, alkyl of 1 to 4 carbons, or alkoxy of 1 to 3 carbons,

X is -O-, -N($R^9$)-, -O-C(=O)-O-, -N($R^9$)-C(=O)-O-, or -O-C(=O)-,

$R^7$ is selected from alkylene of 1 to 6 carbons, arylene of 6 carbons, aralkylene of 9 to 12 carbons, or cycloalkylene of 5 to 7 carbons,

Y is selected from -C(=O)-, -C($R^{10}$)($R^{11}$)-, or -O-C(=O)-,

when p is 1, $R^8$ is selected from acyl of 2 to 10 carbons, aroyl of 7 carbons, t-alkyl of 4 to 6 carbons, t-aralkyl of 9 to 12 carbons, or

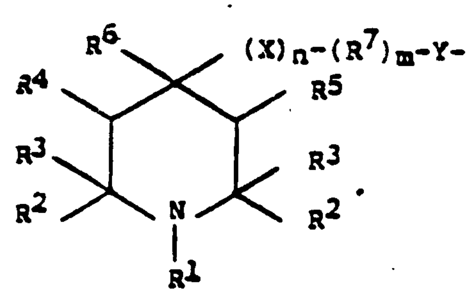

when p is 2, $R^8$ is selected from di-tertiary alkylene of 8 to 10 carbons, di-tertiary aralkylene of 12 carbons, or alkanedioyl of 4 to 6 carbons,

$R^9$ is selected from hydrogen, or alkyl of 1 to 4 carbons,

$R^{10}$ is selected from alkyl of 1 to 4 carbons, aryl of 6 carbons, or cycloalkyl of 6 carbons.

**4.** The compound of claim 3 in which p is 1, n is 0, m is 0, $R^1$, $R^2$, and $R^3$ are methyl, $R^4$, $R^5$ and $R^6$ are hydrogen, Y is -O-C(=O)-, and $R^8$ is t-butyl.

**5.** The compound of claim 3 in which p is 1, n is 1, m is 1, R1, $R^2$, and $R^3$ are methyl, $R^4$, $R^5$, and $R^6$ are hydrogen, X is -O-C(=O)-O-, $R^7$ is $-CH(CH_3)-CH_2$, Y is $-C(CH_3)_2-$, and $R^8$ is t-butyl.

**6.** The compound of claim 3 in which p is 1, n is 0, m is 0, $R^2$ and $R^3$ are methyl, $R^1$, $R^4$, $R^5$, and $R^6$ are hydrogen, Y is -O-C(=O)-, and $R^8$ is t-butyl.

**7.** The compound of claim 3 in which p is 1, n is 1, m is 1, $R^1$, $R^2$, and $R^3$ are methyl, $R^4$, $R^5$, and $R^6$ are hydrogen, X is -O-C(=O)-O-, $R^7$ is $-CH_2-CH_2$, Y is -C(=O)-, and $R^8$ is t-butyl.

**8.** The compound of claim 3 in which p is 1, m is 1, n is 1, $R^2$ and $R^3$ are methyl, $R^1$, $R^4$, $R^5$, and $R^6$ are hydrogen, X is -N($R^9$)-C(=O)-, $R^7$ is 1,4-phenylene additionally connected to $R^9$ in the 2 position, Y is

-C(=O)-, $R^8$ is t-amyl, and $R^9$ is -C(=O)-.

**9.** A process of preparing a homo- or copolymer containing hindered amine light stabilizer groups chemically connected to the backbone of said polymer comprising polymerizing one or more ethylenically unsaturated monomer capable of being polymerized by free radicals in the presence of an initiating amount of the compound of claim 1.

**10.** The stabilized homo- or copolymer product prepared by the process of claim 9.

**11.** A process of preparing a cured polyester containing hindered amine light stabilizer groups chemically connected to said polyester comprising curing an unsaturated polyester resin capable of being cured by free radicals in the presence of a sufficient amount to cure of the compound of claim 1.

**12.** The stabilized cured polyester product prepared by the process of claim 11.

**Patentansprüche**

**1.** Peroxidinitiator enthaltend (eine) sterisch gehinderte lichtstabilisierende Amingruppe(n) mit der Formel:

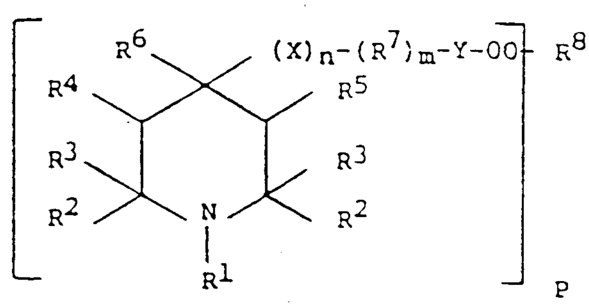

worin p den Wert 1 oder 2 hat,
n und m unabhängig voneinander aus 0 und 1 mit der Maßgabe ausgewählt sind, daß, wenn m den Wert 0 hat, dann n den Wert 0 hat,
$R^1$ aus Wasserstoff, Alkyl mit 1 bis 8 Kohlenstoffatomen, Alkenyl mit 2 bis 8 Kohlenstoffatomen, Alkinyl mit 2 bis 8 Kohlenstoffatomen, Cycloalkyl mit 5 bis 6 Kohlenstoffatomen im Ring, Aralkyl mit 7 bis 15 Kohlenstoffatomen ausgewählt ist,
$R^2$ und $R^3$, die gleich oder verschieden sein können, unabhängig voneinander aus Alkyl mit 1 bis 12 Kohlenstoffatomen, Aryl mit 6 bis 10 Kohlenstoffatomen, Aralkyl mit 7 bis 15 Kohlenstoffatomen ausgewählt sind und $R^2$ und $R^3$ zusammen mit dem Kohlenstoffatom, an das sie angefügt sind, eine gesättigte alicyclische Gruppe mit 4 bis 10 Kohlenstoffatomen, die gegebenenfalls Sauerstoff oder Stickstoff im Ring enthält, bilden können,
$R^4$ und $R^5$, die gleich oder verschieden sein können, unabhängig voneinander aus Wasserstoff, Alkyl mit 1 bis 8 Kohlenstoffatomen, Aryl mit 6 bis 10 Kohlenstoffatomen, Aralkyl mit 7 bis 15 Kohlenstoffatomen ausgewählt sind,
$R^6$ aus Wasserstoff, Alkyl mit 1 bis 8 Kohlenstoffatomen, Alkenyl mit 2 bis 8 Kohlenstoffatomen, Alkinyl mit 2 bis 8 Kohlenstoffatomen, Cycloalkyl mit 5 bis 6 Kohlenstoffatomen, Alkoxy mit 1 bis 8 Kohlenstoffatomen, Alkoxycarbonyl mit 2 bis 7 Kohlenstoffatomen, Aryl mit 6 bis 10 Kohlenstoffatomen, Aralkyl mit 7 bis 15 Kohlenstoffatomen, Acyl mit 1 bis 8 Kohlenstoffatomen, Aroyl mit 7 bis 16 Kohlenstoffatomen, Alkylsulfonyl mit 1 bis 8 Kohlenstoffatomen, Arylsulfonyl mit 6 bis 10 Kohlenstoffatomen, Acyloxy mit 1 bis 7 Kohlenstoffatomen und Aroyloxy mit 6 bis 10 Kohlenstoffatomen ausgewählt ist, und $R^6$ mit $R^7$ unter Bildung einer cyclischen Struktur mit 5 bis 7 Atomen verbunden sein kann,
X aus -O-, -S-, -N($R^9$)-, -C(=O)-, -S(=O)-, $S(=O)_2$-, -O-C(=O)-, -O-S(=O)-, -O-$S(=O)_2$, -N($R^9$)-C(=O)-, -NH-C(=O)-NH-, -O-C(=O)-O- ausgewählt ist,
$R^7$ ein Diradikal aus Alkylen mit 1 bis 20 Kohlenstoffatomen, Arylen mit 6 bis 10 Kohlenstoffatomen, Cycloalkylen mit 3 bis 10 Kohlenstoffatomen, Aralkylen mit 7 bis 20 Kohlenstoffatomen, Alkinylen mit 2

bis 10 Kohlenstoffatomen, Alkadiinylen mit 4 bis 10 Kohlenstoffatomen, Alkenylen mit 3 bis 11 Kohlenstoffatomen, Alkadienylen mit 5 bis 11 Kohlenstoffatomen ausgewählt ist, das gegebenenfalls Sauerstoff, Schwefel oder Stickstoffatome in der Diradikalkette enthält,
Y aus -C(=O)-, S(=O)$_2$-, -C($R^{10}$)($R^{11}$)-, -O-C(=O)-, -N($R^9$)-C(=O)-, -O-C(=O)-C(=O)- ausgewählt ist,
wenn p den Wert 1 hat, $R^8$ aus Wasserstoff, Acyl mit 2 bis 20 Kohlenstoffatomen, Aroyl mit 7 bis 20 Kohlenstoffatomen, t-Alkyl mit 4 bis 12 Kohlenstoffatomen, t-Cycloalkyl mit 4 bis 12 Kohlenstoffatomen, t-Aralkyl mit 9 bis 15 Kohlenstoffatomen, Alkoxycarbonyl mit 2 bis 20 Kohlenstoffatomen, Carbamoyl, Phenylcarbamoyl, Alkylcarbamoyl mit 2 bis 13 Kohlenstoffatomen, Cycloalkylcarbamoyl mit 4 bis 13 Kohlenstoffatomen, alpha-Hydroxyalkyl mit 2 bis 10 Kohlenstoffatomen, alpha-Hydroxycycloalkyl mit 3 bis 10 Kohlenstoffatomen, Alkylsulfonyl mit 4 bis 20 Kohlenstoffatomen, Cycloalkylsulfonyl mit 3 bis 12 Kohlenstoffatomen, t-(Alkoxyalkyl) mit 4 bis 20 Kohlenstoffatomen, t-(Alkoxycycloalkyl) mit 4 bis 20 Kohlenstoffatomen, einwertigem Organomineral, oder

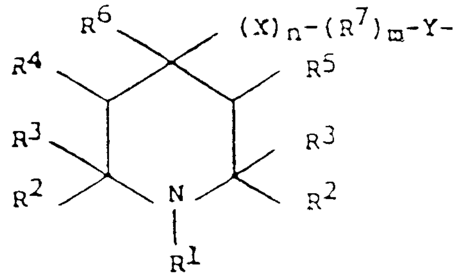

ausgewählt ist,
wenn p den Wert 2 hat, $R^8$ ein Diradikal ist, das aus di-tertiärem Alkylen mit 7 bis 15 Kohlenstoffatomen, di-tertiärem Alkenylen mit 8 bis 16 Kohlenstoffatomen, di-tertiärem Alkinylen mit 8 bis 16 Kohlenstoffatomen, di-tertiärem Aralkylen mit 12 bis 20 Kohlenstoffatomen, Alkandioyl mit 3 bis 12 Kohlenstoffatomen, Aryldicarbonyl mit 8 bis 16 Kohlenstoffatomen oder Aralkyldicarbonyl mit 9 bis 18 Kohlenstoffatomen ausgewählt ist,
$R^9$ aus Wasserstoff, Alkyl mit 1 bis 8 Kohlenstoffatomen, Alkenyl mit 2 bis 8 Kohlenstoffatomen, Alkinyl mit 2 bis 8 Kohlenstoffatomen, Cycloalkyl mit 5 bis 6 Kohlenstoffatomen im Ring, Aryl mit 6 bis 10 Kohlenstoffatomen, Acyl mit 1 bis 8 Kohlenstoffatomen ausgewählt ist, und $R^9$ mit $R^7$ unter Bildung einer cyclischen Struktur mit 5 bis 7 Atomen verbunden sein kann,
$R^{10}$ aus Alkyl mit 1 bis 10 Kohlenstoffatomen, Aryl mit 6 bis 10 Kohlenstoffatomen, Alkinyl mit 2 bis 10 Kohlenstoffatomen, Alkenyl mit 2 bis 8 Kohlenstoffatomen, Cycloalkyl mit 5 bis 6 Kohlenstoffatomen im Ring ausgewählt ist,
$R^{11}$ aus der Definition für $R^{10}$ ausgewählt ist, und $R^{10}$ und $R^{11}$ miteinander mittels einer Alkylendiradikalbrücke mit 4 bis 9 Kohlenstoffatomen verbunden sein können, und wenn $R^8$ für t-Alkyl, t-Cycloalkyl oder t-Aralkyl steht, $R^{11}$ -OO-$R^8$ sein kann,
wobei die Substituenten für $R^1$, $R^2$, $R^3$, $R^4$, $R^5$, $R^6$, $R^7$, $R^8$, $R^9$, $R^{10}$ und $R^{11}$ Halogen, Alkyl mit 1 bis 4 Kohlenstoffatomen, Alkoxy mit 1 bis 4 Kohlenstoffatomen, -C≡N, -OH, Epoxy, Carboxy, Alkoxycarbonyl mit 2 bis 6 Kohlenstoffatomen, Acyloxy mit 1 bis 4 Kohlenstoffatomen, Hydroxymethyl, Hydroxyethyl und Alkylmercapto mit 1 bis 4 Kohlenstoffatomen einschließen.

2. Verbindung nach Anspruch 1, dadurch **gekennzeichnet,** daß $R^1$ aus Wasserstoff, Alkyl mit 1 bis 4 Kohlenstoffatomen, Aralkyl mit 7 bis 9 Kohlenstoffatomen oder Cycloalkyl mit 5 bis 6 Kohlenstoffatomen ausgewählt ist,
$R^2$ und $R^3$ gleich oder verschieden sein können und unabhängig voneinander aus Alkyl mit 1 bis 3 Kohlenstoffatomen, Aralkyl mit 7 bis 10 Kohlenstoffatomen ausgewählt sind, und zusammen mit dem Kohlenstoffatom, an das sie angefügt sind, eine gesättigte alicyclische Gruppe mit 6 Kohlenstoffatomen bilden können,
$R^6$ aus Wasserstoff, Alkyl mit 1 bis 4 Kohlenstoffatomen, Alkoxycarbonyl mit 2 bis 5 Kohlenstoffatomen, Acyloxy mit 1 bis 4 Kohlenstoffatomen, Aryl mit 6 Kohlenstoffatomen oder Alkoxy mit 1 bis 3 Kohlenstoffatomen ausgewählt ist,
X für -O-, -S-, -N($R^9$)-, -O-C(=O)-O-, -N($R^9$)-C(=O)-O-, -O-C(=O)-, -C(=O)- steht,
$R^7$ aus Alkylen mit 1 bis 8 Kohlenstoffatomen, Arylen mit 6 bis 10 Kohlenstoffatomen, Aralkylen mit 8 bis 16 Kohlenstoffatomen oder Cycloalkylen mit 4 bis 8 Kohlenstoffatomen ausgewählt ist,

wenn p den Wert 1 hat, $R^8$ aus Acyl mit 2 bis 10 Kohlenstoffatomen, Aroyl mit 7 bis 10 Kohlenstoffatomen, t-Alkyl mit 4 bis 8 Kohlenstoffatomen, t-Aralkyl mit 9 bis 16 Kohlenstoffatomen, oder

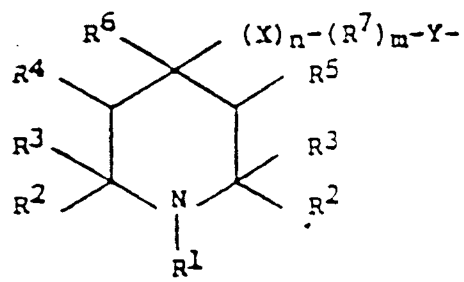

ausgewählt ist,
wenn p den Wert 2 hat, $R^8$ aus di-tertiärem Alkylen mit 8 bis 12 Kohlenstoffatomen, di-tertiärem Aralkylen mit 12 bis 15 Kohlenstoffatomen oder Alkandioyl mit 3 bis 6 Kohlenstoffatomen ausgewählt ist,
$R^9$ aus Wasserstoff, Alkyl mit 1 bis 4 Kohlenstoffatomen, Cycloalkyl mit 6 Kohlenstoffatomen, Aryl mit 6 Kohlenstoffatomen ausgewählt ist, und
$R^{10}$ für Alkyl mit 1 bis 6 Kohlenstoffatomen, Aryl mit 6 bis 10 Kohlenstoffatomen oder Cycloalkyl mit 5 bis 6 Kohlenstoffatomen steht.

**3.** Verbindung nach Anspruch 2, dadurch **gekennzeichnet,** daß $R^1$ aus Wasserstoff oder Alkyl mit 1 bis 4 Kohlenstoffatomen ausgewählt ist,
$R^2$ und $R^3$ gleich oder verschieden sein können und aus Methyl, Ethyl oder Benzyl ausgewählt sind,
$R^4$ und $R^5$ gleich oder verschieden sind und aus Wasserstoff, Methyl, Ethyl oder Phenyl ausgewählt sind,
$R^6$ aus Wasserstoff, Alkyl mit 1 bis 4 Kohlenstoffatomen oder Alkoxy mit 1 bis 3 Kohlenstoffatomen ausgewählt ist,
X für -O-, -N($R^9$)-, -O-C(=O)-O-, -N($R^9$)-C(=O)-O- oder -O-C(=O)- steht,
$R^7$ aus Alkylen mit 1 bis 6 Kohlenstoffatomen, Arylen mit 6 Kohlenstoffatomen, Aralkylen mit 9 bis 12 Kohlenstoffatomen oder Cycloalkylen mit 5 bis 7 Kohlenstoffatomen ausgewählt ist,
Y aus -C(=O)-, -C($R^{10}$)($R^{11}$)- oder -O-C(=O)- ausgewählt ist,
wenn p den Wert 1 hat, $R^8$ aus Acyl mit 2 bis 10 Kohlenstoffatomen, Aroyl mit 7 Kohlenstoffatomen, t-Alkyl mit 4 bis 6 Kohlenstoffatomen, t-Aralkyl mit 9 bis 12 Kohlenstoffatomen oder

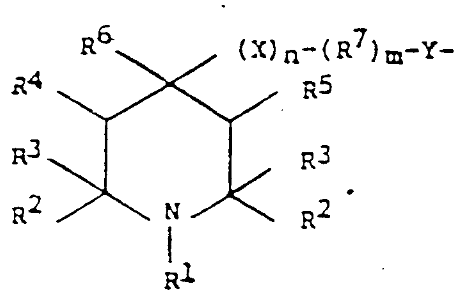

ausgewählt ist,
wenn p den Wert 2 hat, $R^8$ aus di-tertiärem Alkylen mit 8 bis 10 Kohlenstoffatomen, di-tertiärem Aralkylen mit 12 Kohlenstoffatomen oder Alkandioyl mit 4 bis 6 Kohlenstoffatomen ausgewählt ist,
$R^9$ aus Wasserstoff oder Alkyl mit 1 bis 4 Kohlenstoffatomen ausgewählt ist,
$R^{10}$ aus Alkyl mit 1 bis 4 Kohlenstoffatomen, Aryl mit 6 Kohlenstoffatomen oder Cycloalkyl mit 6 Kohlenstoffatomen ausgewählt ist.

**4.** Verbindung nach Anspruch 3, dadurch **gekennzeichnet,** daß p den Wert 1 hat, n den Wert 0 hat, m den Wert 0 hat, $R^1$, $R^2$ und $R^3$ für Methyl stehen, $R^4$, $R^5$ und $R^6$ für Wasserstoff stehen, Y für -O-C-(=O)- steht, und $R^8$ für t-Butyl steht.

**5.** Verbindung nach Anspruch 3, dadurch **gekennzeichnet,** daß p den Wert 1 hat, n den Wert 1 hat, m den Wert 1 hat, $R^1$, $R^2$ und $R^3$ für Methyl stehen, $R^4$, $R^5$ und $R^6$ für Wasserstoff stehen, X für -O-C(=O)-O- steht, $R^7$ für $-CH(CH_3)-CH_2-$ steht, Y für $-C(CH_3)_2-$ steht, und $R^8$ für t-Butyl steht.

**6.** Verbindung nach Anspruch 3, dadurch **gekennzeichnet,** daß p den Wert 1 hat, n den Wert 0 hat, m den Wert 0 hat, $R^2$ und $R^3$ für Methyl stehen, $R^1$, $R^4$, $R^5$ und $R^6$ für Wasserstoff stehen, Y für -O-C(=O)- steht, und $R^8$ für t-Butyl steht.

**7.** Verbindung nach Anspruch 3, dadurch **gekennzeichnet,** daß p den Wert 1 hat, n den Wert 1 hat, m den Wert 1 hat, $R^1$, $R^2$ und $R^3$ für Methyl stehen, $R^4$, $R^5$ und $R^6$ für Wasserstoff stehen, X für -O-C(=O)- steht, $R^7$ für $-CH_2-CH_2-$ steht, Y für -C(=O)- steht, und $R^8$ für t-Butyl steht.

**8.** Verbindung nach Anspruch 3, dadurch **gekennzeichnet,** daß p den Wert 1 hat, m den Wert 1 hat, n den Wert 1 hat, $R^2$ und $R^3$ für Methyl stehen, $R^1$, $R^4$, $R^5$ und $R^6$ für Wasserstoff stehen, X für $-N(R^9)-C(=O)-$ steht, $R^7$ für 1,4-Phenylen steht, das zusätzlich an $R^9$ in 2-Position angeschlossen ist, Y für -C(=O)- steht, $R^8$ für t-Amyl steht, und $R^9$ für -C(=O)- steht.

**9.** Verfahren zur Herstellung eines Homo- oder Copolymeren, das sterisch gehinderte lichtstabilisierende Amingruppen, die chemisch mit dem Gerüst des genannten Polymeren verbunden sind, enthält, dadurch **gekennzeichnet,** daß man ein oder mehrere ethylenisch ungesättigte Monomere, die durch freie Radikale polymerisiert werden können, in Gegenwart einer initiierenden Menge der Verbindung nach Anspruch 1 polymerisiert.

**10.** Stabilisiertes Homo- oder Copolymerprodukt, hergestellt nach dem Verfahren nach Anspruch 9.

**11.** Verfahren zur Herstellung eines gehärteten Polyesters, der sterisch gehinderte lichtstabilisierende Amingruppen, die chemisch mit dem genannten Polyester verbunden sind, enthält, dadurch **gekennzeichnet,** daß man ein ungesättigtes Polyesterharz, das durch freie Radikale gehärtet werden kann, in Gegenwart einer genügenden Menge der Verbindung nach Anspruch 1 zur Härtung härtet.

**12.** Stabilisiertes gehärtetes Polyesterprodukt, hergestellt nach dem Verfahren nach Anspruch 11.

**Revendications**

**1.** Initiateur du type d'un peroxyde contenant un ou plusieurs groupes amino à encombrement stérique photo-stabilisants, répondant à la formule

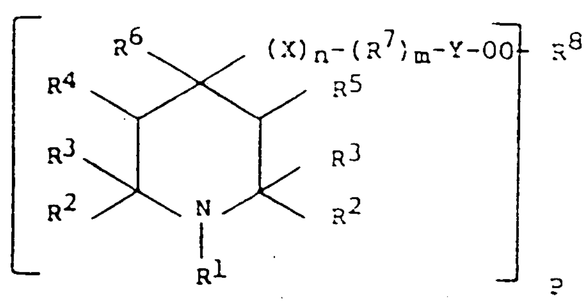

dans laquelle :

p a la valeur 1 ou 2,

n et m sont choisis indépendamment entre les valeurs 0 et 1 sous réserve que lorsque m est égal à 0, n soit alors égal à 0,

$R^1$ est choisi entre l'hydrogène, un groupe alkyle ayant 1 à 8 atomes de carbone, alcényle ayant 2 à 8 atomes de carbone, alcynyle ayant 2 à 8 atomes de carbone, cycloalkyle ayant 5 ou 6 atomes de carbone dans le noyau, aralkyle ayant 7 à 15 atomes de carbone,

$R^2$ et $R^3$ peuvent être identiques ou différents et sont choisis indépendamment entre des groupes alkyle de 1 à 12 atomes de carbone, aryle de 6 à 10 atomes de carbone, aralkyle de 7 à 15 atomes de

carbone et $R^2$ et $R^3$ peuvent former conjointement avec l'atome de carbone auquel ils sont liés un groupe alicyclique saturé de 4 à 10 atomes de carbone, contenant facultativement de l'oxygène ou de l'azote dans le noyau,

$R^4$ et $R^5$ peuvent être identiques ou différents et sont choisis indépendamment entre l'hydrogène, un groupe alkyle de 1 à 8 atomes de carbone, aryle de 6 à 10 atomes de carbone, aralkyle de 7 à 15 atomes de carbone,

$R^6$ est choisi entre l'hydrogène, un groupe de 1 à 8 atomes de carbone, alcényle de 2 à 8 atomes de carbone, alcynyle de 2 à 8 atomes de carbone, cycloalkyle de 5 ou 6 atomes de carbone, alkoxy de 1 à 8 atomes de carbone, alkoxycarbonyle de 2 à 7 atomes de carbone, aryle de 6 à 10 atomes de carbone, aralkyle de 7 à 15 atomes de carbone, acyle de 1 à 8 atomes de carbone, aroyle de 7 à 16 atomes de carbone, alkylsulfonyle de 1 à 8 atomes de carbone, arylsulfonyle de 6 à 10 atomes de carbone, acyloxy de 1 à 7 atomes de carbone et aroyloxy de 6 à 10 atomes de carbone et $R^4$ peut former conjointement avec $R^7$ une structure cyclique de 5 à 7 atomes,

X est choisi entre -O-, -S-, -N($R^9$)-, -C(=O)-, -S(=O)-, $-S(=O)_2-$ -O-C(=O)-, -O-S(=O)-, $-O-S(=O)_2-$, -N($R^9$)-C(=O)-, -NH-C(=O)-NH-, -O-C(=O)-O-,

$R^7$ est un radical divalent choisi entre un radical alkylène de 1 à 20 atomes de carbone, arylène de 6 à 10 atomes de carbone, cycloalkylène de 3 à 10 atomes de carbone, aralkylène de 7 à 20 atomes de carbone, alcynylène de 2 à 10 atomes de carbone, alcadinylène de 4 à 10 atomes de carbone, alcénylène de 3 à 11 atomes de carbone, alcadiénylène de 5 à 11 atomes de carbone, le radical divalent contenant facultativement des atomes d'oxygène, de soufre ou d'azote dans sa chaîne,

Y est choisi entre -C(=O)-, $-S(=O)_2-$, -C($R^{10}$)($R^{11}$)-, -O-C(=O)-, -N($R^9$)-C(=O)-, -O-C(=O)-C(=O)-,

lorsque p est égal à 1, $R^8$ est choisi entre l'hydrogène, un groupe acyle de 2 à 20 atomes de carbone, aroyle de 7 à 20 atomes de carbone, tertioalkyle de 4 à 12 atomes de carbone, tertiocycloalkyle de 4 à 12 atomes de carbone, tertioaralkyle de 9 à 15 atomes de carbone, alkoxycarbonyle de 2 à 20 atomes de carbone, carbamoyle, phénylcarbamoyle, alkylcarbamoyle de 2 à 13 atomes de carbone, cycloalkylcarbamoyle de 4 à 13 atomes de carbone, alphahydroxyalkyle de 2 à 10 atomes de carbone, alpha-hydroxycycloalkyle de 3 à 10 atomes de carbone, alkylsulfonyle de 4 à 20 atomes de carbone, cycloalkylsulfonyle de 3 à 12 atomes de carbone, tertio(alkoxyalkyle) de 4 à 20 atomes de carbone, tertio(alkoxycycloalkyle) de 4 à 20 atomes de carbone, un radical organominéral monovalent ou un groupe

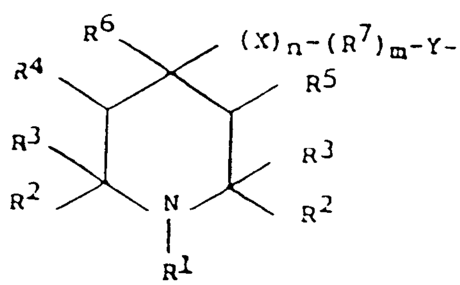

dans lequel p est égal à 2, $R^8$ est un radical divalent choisi entre un radical di-tertioalkylène de 7 à 15 atomes de carbone, di-tertioalcénylène de 8 à 16 atomes, ditertioalcynylène de 8 à 16 atomes de carbone, di-tertioaralkylène de 12 à 20 atomes de carbone, alkanedioyle de 3 à 12 atomes de carbone, aryldicarbonyle de 8 à 16 atomes de carbone ou aralkyldicarbonyle de 9 à 18 atomes de carbone,

$R^9$ est choisi entre l'hydrogène, un groupe alkyle de 1 à 8 atomes de carbone, alcényle de 2 à 8 atomes de carbone, alcynyle de 2 à 8 atomes de carbone, cycloalkyle de 5 ou 6 atomes de carbone dans le noyau, aryle de 6 à 10 atomes de carbone, acyle de 1 à 8 atomes de carbone et $R^9$ peut s'associer avec $R^7$ pour former une structure cyclique de 5 à 7 atomes de carbone,

$R^{10}$ est choisi entre un groupe alkyle de 1 à 10 atomes de carbone, aryle de 6 à 10 atomes de carbone, alcynyle de 2 à 10 atomes de carbone, alcényle de 2 à 8 atomes de carbone, cycloalkyle de 5 ou 6 atomes de carbone dans le noyau,

$R^{11}$ est choisi dans le cadre de la définition de $R^{10}$, et $R^{10}$ et $R^{11}$ peuvent être liés l'un à l'autre au moyen d'un pont formé d'un radical alkylène divalent contenant 4 à 9 atomes de carbone et, lorsque $R^8$ est un groupe tertioalkyle, tertiocycloalkyle ou tertioaralkyle $R^{11}$ peut être un groupe -OO-$R^8$,

les substituants de $R^1$, $R^2$, $R^3$, $R^4$, $R^5$, $R^6$, $R^7$, $R^8$, $R^9$, $R^{10}$ et $R^{11}$ comprennent un halogène, un groupe alkyle de 1 à 4 atomes de carbone, alkoxy de 1 à 4 atomes de carbone, -C≡N, -OH, époxy, carboxy, alkyoxycarbonyle de 2 à 6 atomes de carbone, acylory de 1 à 4 atomes de carbone,

hydroxyméthyle, hydroxyéthyle et alkylmercapto de 1 à 4 atomes de carbone.

**2.** Composé suivant la revendication 1, dans lequel $R^1$ est choisi entre l'hydrogène, un groupe alkyle de 1 à 4 atomes de carbone, aralkyle de 7 à 9 atomes de carbone ou cycloalkyle de 5 ou 6 atomes de carbone,

$R^2$ et $R^3$ peuvent être identiques ou différents et sont choisis indépendamment entre un groupe alkyle de 1 à 3 atomes de carbone, aralkyle de 7 à 10 atomes de carbone et peuvent former conjointement avec le carbone auquel ils sont liés un groupe alicyclique saturé de 6 atomes

$R^6$ est choisi entre l'hydrogène, un groupe alkyle de 1 à 4 atomes de carbone, alkoxycarbonyle de 2 à 5 atomes de carbone, acyloxy de 1 à 4 atomes de carbone, aryle de 6 atomes de carbone ou alkoxy de 1 à 3 atomes de carbone,

X représente -O-, -S-, -N($R^9$)-, -O-C(=O)-O-, -N($R^9$)-C(=O)-O-, -O-C(=O)-, -C(=O)-,

$R^7$ est choisi entre un groupe alkylène de 1 à 8 atomes de carbone, arylène de 6 à 10 atomes de carbone, aralkylène de 8 à 16 atomes de carbone ou cycloalkylène de 4 à 8 atomes de carbone,

lorsque p est égal à 1, $R^8$ est choisi entre un groupe acyle de 2 à 10 atomes de carbone, aroyle de 7 à 10 atomes de carbone, tertioalkyle de 4 à 8 atomes de carbone, tertioaralkyle de 9 à 16 atomes de carbone, ou

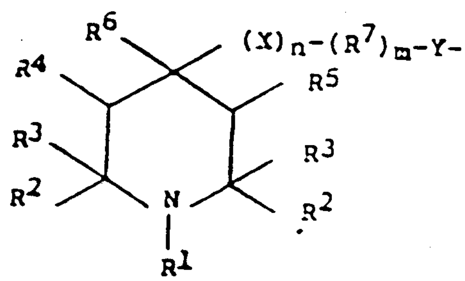

lorsque p est égal à 2, $R^8$ est choisi entre un groupe di-tertioalkylène de 8 à 12 atomes de carbone, di-tertioaralkylène de 12 à 15 atomes de carbone ou alcanedioyle de 3 à 6 atomes de carbone,

$R^9$ est choisi entre l'hydrogène, un groupe alkyle de 1 à 4 atomes de carbone, cycloalkyle de 6 atomes de carbone, aryle de 6 atomes de carbone, et

$R^{10}$ est un groupe alkyle de 1 à 6 atomes de carbone, aryle de 6 à 10 atomes de carbone ou cycloalkyle de 5 ou 6 atomes de carbone.

**3.** Composé suivant la revendication 2, dans lequel $R^1$ est choisi entre l'hydrogène et un groupe alkyle de 1 à 4 atomes de carbone,

$R^2$ et $R^3$ peuvent être identiques ou différents et sont choisis entre un groupe méthyle, éthyle ou benzyle,

$R^4$ et $R^5$ sont identiques ou différents et sont choisis entre l'hydrogène , un groupe méthyle, éthyle ou phényle,

$R^6$ est choisi entre l'hydrogène, un groupe alkyle de 1 à 4 atomes de carbone ou alkoxy de 1 à 3 atomes de carbone,

X représente -O-, -N($R^9$)-, -O-C(=O)-O-, -N($R^9$)-C(=O)-O- ou -O-C(=O)-,

$R^7$ est choisi entre un groupe alkylène de 1 à 6 atomes de carbone, arylène de 6 atomes de carbone, aralkylène de 9 à 12 atomes de carbone ou cycloalkylène de 5 à 7 atomes de carbone,

Y est choisi entre un groupe -C(=O)-, -C($R^{10}$)($R^{11}$)- ou -O-C(=O)-,

lorsque p est égal à 1, $R^8$ est choisi entre un groupe acyle de 2 à 10 atomes de carbone, aroyle de 7 atomes de carbone, tertioalkyle de 4 à 6 atomes de carbone, tertioaralkyle de 9 à 12 atomes de carbone ou

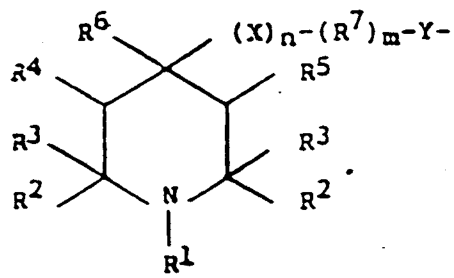

lorsque p est égal à 2, $R^8$ est choisi entre un groupe ditertioalkylène de 8 à 10 atomes de carbone, di-tertioaralkylène de 12 atomes de carbone ou alcanedioyle de 4 à 6 atomes de carbone,

$R^9$ est choisi entre l'hydrogène, ou un groupe alkyle de 1 à 4 atomes de carbone,

$R^{10}$ est choisi entre un groupe alkyle de 1 à 4 atomes de carbone, aryle de 6 atomes de carbone ou cycloalkyle de 6 atomes de carbone.

**4.** Composé suivant la revendication 3, dans lequel p est égal à 1, n est égal à 0, m est égal à 0, $R^1$, $R^2$ et $R^3$ sont des groupes méthyle, $R^4$, $R^5$ et $R^6$ sont de l'hydrogène, Y est un groupe -O-C(=O)-, et $R^8$ est un groupe tertiobutyle.

**5.** Composé suivant la revendication 3, dans lequel p est égal à 1, n est égal à 1, m est égal à 1, $R^8$, $R^2$ et $R^3$ sont des groupes méthyle, $R^4$, $R^5$ et $R^6$ sont de l'hydrogène, X est groupe -O-C(=O)-O-, $R^7$ est un groupe $-CH(CH_3)-CH_2-$, Y est un groupe $-C(CH_3)_2-$, et $R^8$ est un groupe tertiobutyle.

**6.** Composé suivant la revendication 3, dans lequel p est égal à 1, n est égal à 0, m est égal à 0, $R^2$ et $R^3$ sont des groupes méthyle, $R^1$, $R^4$, $R^5$ et $R^6$ sont de l'hydrogène, Y est groupe -O-C(=O)- et $R^8$ est un groupe tertiobutyle.

**7.** Composé suivant la revendication 3, dans lequel p est égal à 1, n est égal à 1, m est égal à 1, $R^1$, $R^2$ et $R^3$ sont des groupes méthyle, $R^4$, $R^5$ et $R^6$ sont de l'hydrogène, X est groupe -O-C(=O)-, $R^7$ est un groupe $-CH_2-CH_2-$, Y est un groupe -C(=O)-, et $R^8$ est un groupe tertiobutyle.

**8.** Composé suivant la revendication 3, dans lequel p est égal à 1, m est égal à 1, n est égal à 1, $R^2$ et $R^3$ sont des groupes méthyle, $R^1$, $R^4$, $R^5$ et $R^6$ sont de l'hydrogène, X est un groupe $-N(R^9)-C(=O)-$ et $R^7$ est un groupe 1,4-phénylène lié en outre à $R^9$ en position 2, Y est un groupe -C(=O)-, $R^8$ est un groupe tertioamyle et $R^9$ est un groupe -C(=O)-.

**9.** Procédé de production d'un homopolymère ou d'un copolymère contenant des groupes amino à encombrement stérique photostabilisants, en liaison chimique avec le squelette dudit polymère, qui consiste à polymériser un ou plusieurs monomères à non-saturation éthylénique capables d'être polymérisés par des radicaux libres en présence d'une quantité servant d'initiateur du composé de la revendication 1.

**10.** Produit homopolymère ou copolymère stabilisé préparé par le procédé suivant la revendication 9.

**11.** Procédé de production d'un polyester mûri contenant des groupes amino à encombrement stérique photostabilisants attachés audit polyester, qui consiste à conduire la maturation d'une résine polyester non saturé capable d'être mûrie par des radicaux libres en présence d'une quantité, suffisante pour la maturation, du composé suivant la revendication 1.

**12.** Produit polyester mûri stabilisé, obtenu par le procédé suivant la revendication 11.